# EUROPEAN PATENT APPLICATION

(11) **EP 4 310 498 A1**
(43) Date of publication of application: **24.01.2024**
(21) Application number: 21931778.1
(22) Date of filing: 28.12.2021
(51) Int. Cl.: G01N 33/543

(54) **CONJUGATE FORMATION METHOD, SUBSTANCE OF INTEREST MEASUREMENT METHOD, AND KIT**

(30) Priority: 17.03.2021 JP 2021043965; 17.03.2021 JP 2021044004
(71) Applicant: JVCKenwood Corporation, Yokohama-shi, Kanagawa 2210022 (JP)
(72) Inventor: KAWAKAMI Tatsuya, Yokohama-shi, Kanagawa 221-0022 (JP); ONO Masayuki, Yokohama-shi, Kanagawa 221-0022 (JP); TSUJITA Koji, Yokohama-shi, Kanagawa 221-0022 (JP); HORIKOSHI Katsue, Yokohama-shi, Kanagawa 221-0022 (JP); HASEGAWA Yuichi, Yokohama-shi, Kanagawa 221-0022 (JP); ITONAGA Makoto, Yokohama-shi, Kanagawa 221-0022 (JP)
(74) Representative: Pritzlaff, Stefanie Lydia
(86) International application number: PCT/JP2021/048974
(87) International publication number: WO 2022/196036

(57) **Abstract**

A method for forming a conjugate of a substance of interest and a magnetic particle to which a specific binding substance that has an ability to specifically bind to the substance of interest has been bound, the method including a step (A1) of reacting the substance of interest and the magnetic particles in a reaction solution, in which the step (A 1 ) includes increasing momentum of the magnetic particles in the reaction solution by changing the magnetic field that is exerted on the magnetic particles.

## Description

### [Technical Field]

The present invention relates to a conjugate formation method, a substance of interest measurement method, and a kit. The conjugate is composed of a substance of interest and a magnetic particle. Alternatively, the conjugate is composed of a substance of interest, a magnetic particle to which a first specific binding substance that has an ability to specifically bind to the substance of interest has been bound, and a second specific binding substance that has an ability to specifically bind to the substance of interest and has been bound to an inner wall of a reaction container.

Priority is claimed on Japanese Patent Application No. 2021-043965 and Japanese Patent Application No. 2021-044004, filed in Japan on March 17, 2021, the contents of which are incorporated herein by reference.

### [Background Art]

A method in which a substance of interest is captured with a solid-phase support to which an antibody or the like that specifically binds to the substance of interest (capture molecule) has been fixed by an immunochemical analysis method represented by an antigen-antibody reaction and the substance of interest is labeled with nanoparticles or the like to which an antibody or the like that specifically binds to the substance of interest (labeled particles) has been fixed is known (for example, Patent Document 1). The substance of interest and the labeled particles bind to each other depending on the probability of the substance of interest and the labeled particles encountering each other in a reaction solution due to Brownian motion. Therefore, approximately two hours is required for the binding reaction to be saturated. The time necessary for the saturation of the binding reaction also relies on the concentration of each of the substance of interest and the labeled particles. In order to highly efficiently perform the binding reaction within a short period of time, it is necessary to set the concentration of the labeled particles to be as high as 100 or more times that of the substance of interest. Therefore, a large amount of the labeled particles is required, which is not economical. In addition, even when the concentration of the labeled particles is set to be high, since the binding reaction occurs with a probability attributed to Brownian motion, the limit of the time necessary for the saturation of the binding reaction is approximately one hour (approximately halved). Furthermore, even in a reaction of capturing the substance of interest with the capture molecule fixed to the solid-phase support, approximately the same period of time is required for the reaction to be saturated.

Incidentally, in an ELISA method, which is a typical immunochemical analysis method represented by an antigen-antibody reaction, a substance of interest is captured with a capture antibody fixed to a solid-phase surface of a microplate or the like. After that, an unreacted substance in a sample is washed away and removed, and the substance of interest is labeled with an antibody for labeling a substance of interest labeled with an enzyme or the like (labeled body). Next, an unreacted labeled body is washed away and removed, and then the labeled body is detected by a chromogenic reaction or the like of the enzyme, thereby determining the quantity of the substance of interest. The ELISA method includes two reaction steps, that is, a reaction between the substance of interest and the capture antibody and a reaction between the substance of interest and the labeled body, and is thus referred to as a two-step method. A one-step method where the capture and labeling of a substance of interest is completed in one step can be performed within a short period of time, but the quantity determination property becomes poor. In the ELISA method, one hour or longer is required for the reaction in each step to be saturated. Therefore, in the two-step method, a minimum of approximately five hours is required until measurement is completed.

### [Citation List]

### [Patent Literature]

[Patent Document 1]
Japanese Unexamined Patent Application, First Publication No. 2017-207289

### [Summary of Invention]

### [Technical Problem]

As long as the binding reaction between a substance of interest and labeled particles can be performed within a short period of time, more rapid quantity determination of the substance of interest becomes possible.

In addition, as long as the binding reaction between a substance of interest and labeled particles and the binding reaction between the substance of interest and a capture molecule can be performed within a short period of time, more rapid quantity determination of the substance of interest becomes possible.

Therefore, an objective of the present invention is to provide a formation method of a conjugate of a substance of interest and a magnetic particle in which the reaction time between the substance of interest and the magnetic particle can be reduced, a substance of interest measurement method, and a kit that is used in the methods.

In addition, another objective of the present invention is to provide a conjugate formation method in which the reaction time among a substance of interest, magnetic particles to which a first specific binding substance has been bound, and a second specific binding substance bound to a reaction container can be reduced, a substance of interest measurement method, and a kit that is used in the methods.

### [Solution to Problem]

The present invention includes the following aspects.
[1] A method for forming a conjugate of a substance of interest and a magnetic particle to which a specific binding substance that has an ability to specifically bind to the substance of interest has been bound, the method including a step (A1) of reacting the substance of interest and the magnetic particles in a reaction solution, in which the step (A1) includes increasing momentum of the magnetic particles in the reaction solution by changing the magnetic field that is exerted on the magnetic particles.
[2] The method according to [1], in which the step (A1) further includes stirring or shaking the reaction solution containing the substance of interest and the magnetic particles.
[3] A method for measuring the substance of interest, the method including a step (a1) of forming the conjugates by the method according to [1] or [2] and a step (b1) of determining the quantity of the substance of interest by determining the quantity of the magnetic particles that form the conjugates.
[4] A kit that is used in the method according to [1] of [2], the kit including magnetic particles to which a specific binding substance that has an ability to specifically bind to a substance of interest has been bound, a reaction container in which the substance of interest and the magnetic particles are reacted, and a magnet.
[5] A kit that is used in the method according to [1] or [2], the kit including magnetic particles to which a specific binding substance that has an ability to specifically bind to a substance of interest can be bound, a reaction container in which the substance of interest and the magnetic particles are reacted, and a magnet.
[6] A method for forming a conjugate of a substance of interest, a magnetic particle to which a first specific binding substance that has an ability to specifically bind to the substance of interest has been bound, and a second specific binding substance that has ability to specifically bind to the substance of interest and has been bound to an inner wall of a reaction container, the method including a step (A2) of performing a binding reaction between the substance of interest and the magnetic particle and a binding reaction between the substance of interest and the second specific binding substance in a reaction solution accommodated in the reaction container and containing the substance of interest and the magnetic particles, in which momentum of the magnetic particles in the reaction solution is increased by changing the magnetic field that is exerted on the magnetic particles.
[7] The method according to [6], in which the step (A2) further includes stirring or shaking the reaction solution.
[8] A method for measuring the substance of interest, the method including a step (a2) of forming the conjugates by the method according to [6] or [7] and a step (b2) of determining the quantity of the substance of interest by determining the quantity of the magnetic particles that form the conjugates.
[9] A kit that is used in the method according to [6] of [7], the kit including magnetic particles to which a first specific binding substance that has an ability to specifically bind to a substance of interest has been bound, a reaction container in which a second specific binding substance that has an ability to specifically bind to the substance of interest has been bound to the inner wall of the reaction container, and a magnet.
[10] A kit that is used in the method according to [6] or [7], the kit including magnetic particles to which a first specific binding substance that has an ability to specifically bind to the substance of interest can be bound, a reaction container in which a second specific binding substance that has an ability to specifically bind to the substance of interest has been bound to the inner wall of the reaction container, and a magnet.

### [Advantageous Effects of Invention]

According to the present invention, a formation method of a conjugate of a substance of interest and a magnetic particle in which the reaction time between the substance of interest and the magnetic particle can be reduced, a substance of interest measurement method, and a kit that is used in the methods are provided.

In addition, according to the present invention, a conjugate formation method in which the reaction time among a substance of interest, a magnetic particle to which a first specific binding substance has been bound, and a second specific binding substance bound to a reaction container can be reduced, a substance of interest measurement method, and a kit that is used in the methods are provided.

### [Brief Description of Drawings]

FIG. 1 is a view schematically describing an example of a state where a magnetic field is exerted in a step (A1) of a method according to one embodiment of the present invention.
FIG. 2 is a view schematically describing an example of a state where the magnetic field is not exerted in the step (A1) of the method according to the embodiment of the present invention.
FIG. 3 is a view showing a state where magnetic particles including conjugates of a substance of interest and a magnetic particle have been captured on the bottom surface of a reaction container using a magnet.
FIG. 4 is a view schematically describing an example of a state where a magnetic field is exerted in a step (A2) of the method according to the embodiment of the present invention.
FIG. 5 is a view schematically describing an example of a state where the magnetic field is not exerted in the step (A2) of the method according to the embodiment of the present invention.
FIG. 6 is a view schematically describing an example of a reaction container after the step (A2) of the method according to the embodiment of the present invention.
FIG. 7 shows the results of the quantities of exosomes determined by a method according to one embodiment of the present invention (Example 1) and a conventional method (Comparative Example 1). For the determination of the quantities of exosomes, ExoCounter (registered trademark) (JVCKENWOOD Corporation) was used. In the drawing, "Exosome" indicates a result measured using an exosome-containing sample, and "Blank" indicates a result measured using an exosome-free sample.
FIG. 8 shows the results of the quantities of exosomes determined by the method according to the embodiment of the present invention under different reaction conditions (the magnetic sweep time, the shaking and stirring time, and the number of cycles) between the exosome and the magnetic particles. For the determination of the quantities of exosomes, ExoCounter was used. In the drawing, "Exosome" indicates a result measured using an exosome-containing sample, and "Blank" indicates a result measured using an exosome-free sample.
FIG. 9 shows the relationship between the total reaction time and the detection rate in a case where the quantities of exosomes have been determined by the method according to the embodiment of the present invention. For the determination of the quantities of exosomes, ExoCounter was used.
FIG. 10 shows the relationship between the amount of a substance of interest and the count with ExoCounter in a case where the quantities of exosomes have been determined by the method according to the embodiment of the present invention.

### [Description of Embodiments]

Hereinafter, an embodiment of the present invention will be described in detail while referring to drawings in some cases. In the drawings, the same or equivalent portions will be given the same or corresponding reference signs and will not be described again. The dimensional ratio in each drawing may be exaggerated in some part for description and does not always match the actual dimensional ratio.

The term "comprise" means that configuration elements other than a configuration element of interest may be included. The term "consist of" means that configuration elements other than a configuration element of interest are not included. The term "consist essentially of" means that configuration elements other than a configuration element of interest are not included in an aspect where a special function is exhibited (an aspect where the effect of the invention is completely lost or the like). In the present specification, in a case where "comprise" is used for description, the case includes the "consist of" aspect and the "consist essentially of' aspect.

Proteins, peptides, nucleic acids, exosomes and cells can be isolated ones. "Being isolated" means a natural state or a state where a substance of interest has been isolated from other components. An "isolated" substance may substantially not contain other components. "Substantially not containing other components" means that the content of other components in an isolated component is small enough to be ignored. The content of other components that are contained in the isolated component can be, for example, 10 mass% or less, 5 mass% or less, 4 mass% or less, 3 mass% or less, 2 mass% or less, 1 mass% or less, 0.5 mass% or less, or 0.1 mass% or less. Proteins, peptides, nucleic acids, exosomes and cells described in the present specification can be isolated proteins, isolated peptides, isolated nucleic acids, isolated exosomes and isolated cells.

"Specific binding substance" means a substance that has an ability specifically bind to a specific biomolecule. "Having an ability to specifically bind to" means having a high binding affinity to a specific biomolecule but having only an extremely low binding affinity to other biomolecules. It is preferable that a specific binding substance highly specifically bind to a specific biomolecule, but rarely bind to other biomolecules.

Examples of the combination of a biomolecule and a specific binding substance include a peptide or a protein and an antibody; a nucleic acid and a nucleic acid including a complementary sequence to a partial sequence of the nucleic acid; a ligand and a receptor thereof; an enzyme and a substrate, inhibitor or cofactor thereof; a sugar chain and a lectin; a peptide, a protein, a nucleic acid or the like and an aptamer; a transcriptional regulatory sequence part of a nucleic acid and a transcriptional regulator; and the like, but the combination is not limited thereto.

"Antibody" means an immunoglobulin having an antigen binding activity. The antibody does not need to be an intact antibody and may be an antigen binding fragment as long as the antibody has an antigen binding activity. In the present specification, the term "antibody" includes antigen binding fragments. "Antigen binding fragment" is a polypeptide including a part of an antibody and maintaining the antigen binding property of the original antibody. The antigen binding fragment preferably includes all of six complementarity determining regions (CDRs) of the original antibody. That is, the antigen binding fragment preferably includes all of CDR1, CDR2, and CDR3, which are heavy-chain variable regions, and CDR1, CDR2, and CDR3, which are light-chain variable regions. Examples of the antigen binding fragment include Fab, Fab', F(ab')₂, a variable region fragment (Fv), disulfide-bonded Fv, single-chain Fv (scFv), sc (Fv)₂, and the like.

Antibodies may be derived from any organism. Examples of organisms from which antibodies are derived include mammals (humans, mice, rats, rabbits, horses, cows, pigs, monkeys, dogs, and the like), birds (chickens and ostriches), and the like, but the organisms are not limited thereto.

Antibodies may be immunoglobulins of any classes and subclasses. In addition, antibodies may be monoclonal antibodies or may be polyclonal antibodies, but are preferably monoclonal antibodies.

Antibodies can be produced by a well-known method such as an immunization method, a hybridoma method, or a phage display method.

<Conjugate formation method (1)>

In one embodiment, the present invention provides a method for forming a conjugate of a substance of interest and a magnetic particle to which a specific binding substance that has an ability to specifically bind to the substance of interest has been bound. The method of the present embodiment includes a step (A1) of reacting the substance of interest and the magnetic particles in a reaction solution. The step (A1) includes increasing the momentum of the magnetic particles in the reaction solution by changing the magnetic field that is exerted on the magnetic particles.

"Substance of interest" means a substance of interest used to form a conjugate with a magnetic particle. The substance of interest is not particularly limited, but there needs to be a substance that has an ability to specifically bind to the substance of interest. Examples of the substance of interest include an exosome, a peptide, a protein, a nucleic acid, a sugar chain, a lectin, a lipid, and the like, but the substance of interest is not limited thereto.

The substance of interest may be, for example, a substance that is contained in a biological sample or the like. The biological sample is not particularly limited, and a sample collected from a biological body can be used. Examples of the biological sample include body fluid samples such as blood, serum, plasma, saliva, urine, tears, sweat, milk, nasal discharge, semen, pleural effusion, gastrointestinal secretions, cerebrospinal fluid, interstitial fluid, and lymph, cell debris samples, cell extract samples, and the like, but the biological sample is not limited thereto. The biological sample may be an extracted or concentrated fraction of a specific biological substance. For example, in a case where the substance of interest is an exosome, the biological sample may be an exosome fraction concentrated from a body fluid sample by ultracentrifuge or the like.

The specific binding substance can be selected as appropriate depending on the substance of interest. For example, in a case where the substance of interest is an exosome, examples of the specific binding substance include an antibody that specifically binds to a membrane protein of the exosome, a lectin that specifically binds to a sugar chain of the exosome, and the like. The membrane protein or sugar chain of the exosome may be a membrane protein or sugar chain that is widely expressed in exosomes or may be a membrane protein or sugar chain that is expressed only in an exosome discharged from a specific cell (for example, a cancer cell). Examples of the membrane protein that is widely expressed in exosomes (hereinafter, also referred to as "wide exosome membrane protein") include CD9, CD63, CD81, and the like.

"Magnetic particle" means a particle exhibiting magnetism. The magnetic particles are not particularly limited as long as the magnetic particles are attracted to magnets. The material of the magnetic particles is not particularly limited, and examples thereof include iron, cobalt, zinc, nickel, compounds, oxides, and alloys thereof, and the like. The particle diameters of the magnetic particles are not particularly limited, but are preferably so small that the reaction between the substance of interest and the magnetic particles is not impaired. The particle diameters of the magnetic particles are, for example, preferably 1000 nm or less, more preferably 1 to 500 nm, still more preferably 10 to 300 nm, and particularly preferably 10 to 200 nm. The magnetic particles are preferably nanoparticles having nanosized (1 to 1000 nm) particle diameters.

In the method of the present embodiment, magnetic particles to which a specific binding substance has been bound are used as the magnetic particles. The specific binding substance and the magnetic particles can be bound by a well-known method depending on the kind of the specific binding substance. Examples of a method for binding the specific binding substance to the magnetic particles include a method in which physical adsorption is used, a method in which magnetic particle surfaces are modified with a functional group that reacts with a functional group of the specific binding substance (for example, modified with an amino group, modified with a hydroxyl group, modified with a carboxy group, modified with a succinimide group, or the like), a method in which a biotin-avidin binding is used, and the like.

### (Step (A1))

The step (A1) is a step of reacting the substance of interest and the magnetic particles in a reaction solution. The step (A1) includes increasing the momentum of the magnetic particles in the reaction solution by changing the magnetic field that is exerted on the magnetic particles.

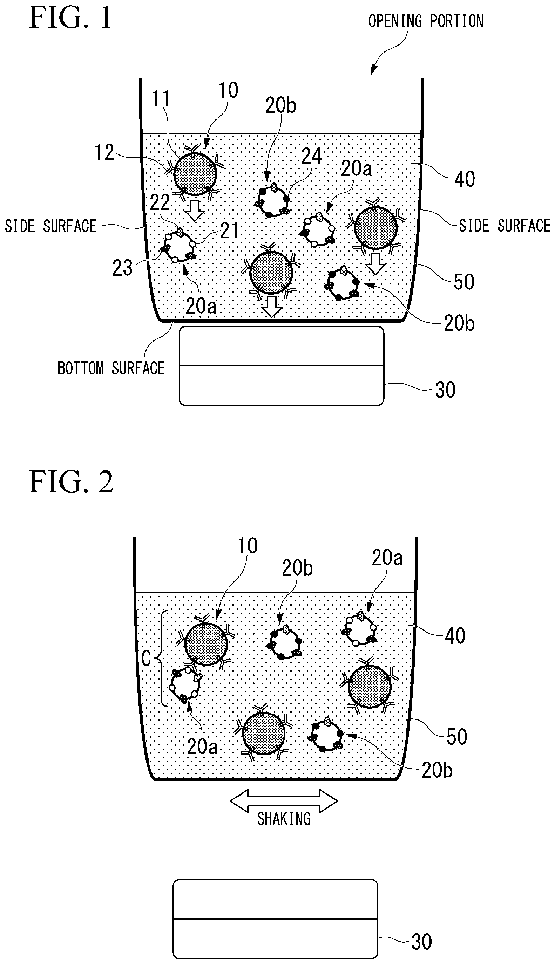

FIG. 1 is a view for schematically describing an example of the step (A1). In an example of FIG. 1, a case where the substance of interest is an exosome is shown. In FIG. 1, a reaction solution 40 has been put into a reaction container 50. The reaction solution 40 contains exosomes 20a, exosomes 20b, and magnetic particles 11 to which a specific binding substance 12 has been bound (hereinafter, the magnetic particles 11 to which a specific binding substance 12 has been bound will be referred to as "magnetic particles 10"). The exosome 20a contains exosome membrane proteins 21, 22 and 23. The exosome 20b contains exosome membrane proteins 22, 23 and 24. The specific binding substance 12 is, for example, an antibody that specifically binds to the exosome membrane protein 21. Therefore, the magnetic particles 10 bind to the exosomes 20a through the specific binding substance 12, but do not bind to the exosomes 20b.

In the reaction solution containing the substance of interest (the exosomes 20a and 20b) and the magnetic particles 10, a change in the magnetic field that is exerted on the magnetic particles 10 increases the momentum of the magnetic particles 10 due to the exertion of the magnetic field. "A change in the magnetic field that is exerted on the magnetic particles" may be a change in the intensity of the magnetic field that is exerted on the magnetic particles or may be a change in the direction of the magnetic field that is exerted on the magnetic particles. "A change in the magnetic field that is exerted on the magnetic particles" is preferably a change in the intensity of the magnetic field that is exerted on the magnetic particles. For example, in the example of FIG. 1, when a magnet 30 comes close to the bottom surface of the reaction container 50, the magnetic field that is exerted on the magnetic particles 10 becomes strong. This makes the magnetic particles 10 attracted in a direction toward the magnet 30, which is the generation source of the magnetic field, and thus makes it possible to increase the momentum of the magnetic particles 10.

The increase in the momentum of the magnetic particles 10 increase the encounter probability of the magnetic particle 10 and the exosome 20a. This makes it possible to accelerate the binding reaction between the exosome 20a and the magnetic particle 10 (more specifically, the binding reaction between the exosome membrane protein 21 and the specific binding substance 12). Therefore, the formation of conjugates C of the exosome 20a and the magnetic particle 10 is accelerated (refer to FIG. 2).

The magnet 30 may be brought close in the side surface direction of the reaction container 50. For example, in a case where there are no other reaction containers in the side surface direction of the reaction container 50, the magnet 30 can be brought close in the side surface direction of the reaction container 50. In addition, the magnet 30 may be brought close in the opening portion direction of the reaction container 50.

Alternatively, the position of the magnet 30 may be sequentially switched between any two or more positions selected in the bottom surface direction, the side surface direction, and the opening portion direction. A change in the position of the magnet 30 makes it possible to change the direction of the magnetic field that is exerted on the magnetic particles 10. Since the magnetic particles 10 are attracted in a direction toward the magnet 30 depending on the change in the position of the magnet 30, the momentum of the magnetic particles 10 can be increased.

The magnet 30 may be a permanent magnet or may be an electromagnet. In a case where the magnet 30 is an electromagnet, the magnetic field can be changed by changing the amount of electricity conducted. Therefore, it is not necessary to move the magnet 30 with respect to the reaction solution 40.

In a case where the magnet 30 is a permanent magnet, the magnet 30 is preferably present outside the reaction container 50. In a case where the magnet 30 is an electromagnet, the magnet 30 may be present outside the reaction container 50 or may be integrated with the bottom surface or side surface of the reaction container 50.

In the step (A1), after the magnetic field that is exerted on the magnetic particles 10 is changed, it is preferable to maintain the state after the change for a certain period of time. It is preferable to alternately repeat a state where the magnetic field attributed to the magnet 30 is exerted on the magnetic particles 10 (hereinafter, also referred to as "magnetic field exertion state") and a state where the magnetic field attributed to the magnet 30 is not exerted on the magnetic particles 10 (hereinafter, also referred to as "no magnetic field state"). From the viewpoint of improvement in the formation efficiency of the conjugates C and reduction in non-specific binding, the continuation time of the magnetic field exertion state is preferably 30 seconds or shorter, more preferably 20 seconds or shorter, still more preferably 10 seconds or shorter, and particularly preferably six seconds or shorter or three seconds or shorter. The lower limit of the continuation time of the magnetic field exertion state is not particularly limited as long as the momentum of the magnetic particles 10 can be increased during that time. Examples of the lower limit of the continuation time of the magnetic field exertion state include 0.5 seconds or longer, one second or longer, 1.2 seconds or longer, or 1.5 seconds or longer.

In a case where the magnet 30 is a permanent magnet, the magnetic field exertion state may be continued by continuing a state where the magnet 30 has been brought close to the bottom surface (or side surface or opening portion) of the reaction container 50. In a case where the magnet 30 is an electromagnet, the magnetic field exertion state may be continued by continuing a state where the electricity has been conducted.

The continuation time of the no magnetic field state is not particularly limited; however, from the viewpoint of accelerating the formation of the conjugates C, the continuation time can be set to, for example, 60 seconds or shorter and is preferably 40 seconds or shorter, more preferably 30 seconds or shorter, still more preferably 20 seconds or shorter, and particularly preferably 15 seconds or shorter or 10 seconds or shorter. The lower limit of the continuation time of the no magnetic field state is not particularly limited, and, from the viewpoint of reducing non-specific binding, examples thereof include 0.5 seconds or longer, one second or longer, two seconds or longer, three seconds or longer, or five seconds or longer.

In a case where the magnet 30 is a permanent magnet, the no magnetic field state may be continued by continuing a state where the magnet 30 has been distanced from the bottom surface (or side surface or opening portion) of the reaction solution 40 by a distance at which the magnetic field attributed to the magnet 30 is not exerted. In a case where the magnet 30 is an electromagnet, the no magnetic field state may be continued by continuing a state where the electricity is not conducted.

The intensity of the magnetic force of the magnet 30 is not particularly limited as long as the momentum of the magnetic particles 10 can be increased. Examples of the intensity of the magnetic force of the magnet 30 include magnetic flux densities of 0.1 to 0.6 tesla.

The step (A1) may further include stirring or shaking the reaction solution containing the substance of interest and the magnetic particles (FIG. 2). The stirring or shaking makes it possible to improve the formation efficiency of the conjugates C. The stirring or shaking can be performed using, for example, a shaker or the like. When the reaction solution is shaken or stirred in the magnetic field exertion state, the reaction solution 40 swirls, and there are cases where the magnetic particles 10 are not evenly distributed. Therefore, the reaction solution is preferably shaken or stirred in the no magnetic field state as shown in FIG. 2.

The shaking or stirring rate is not particularly limited and can be, for example, 100 to 3000 rpm.

In a case where one cycle consists of one magnetic field exertion state and one no magnetic field state, the number of cycles of the magnetic field exertion state and the no magnetic field state is preferably 10 cycles or more, 15 cycles or more, 20 cycles or more, 25 cycles or more, 30 cycles or more, 40 cycles or more, 50 cycles or more, 60 cycles or more, 70 cycles or more, 80 cycles or more, 90 cycles or more, or 100 cycles or more. The upper limit of the number of cycles is not particularly limited, but can be set to, for example, 500 cycles or less, 400 cycles or less, 300 cycles or less, 200 cycles or less, or 150 cycles or less from the viewpoint of reducing the reaction time.

Any of the magnetic field exertion state and the no magnetic field state may be performed earlier. For example, at the time of beginning the step (A1), the step may begin in the magnetic field exertion state, next, the no magnetic field state may follow, and then, the magnetic field exertion state and the no magnetic field state may be alternately repeated. Alternatively, at the time of beginning the step (A1), the step may begin in the no magnetic field state, next, the magnetic field exertion state may follow, and then, the no magnetic field state and the magnetic field exertion state may be alternately repeated.

The total reaction time including both the reaction time in the magnetic field exertion state and the reaction time in the no magnetic field state can be set to, for example, 100 seconds or longer, 200 seconds or longer, 300 seconds or longer, 400 seconds or longer, 500 seconds or longer, 600 seconds or longer, or 650 seconds or longer. The upper limit of the total reaction time is not particularly limited, and can be set to, for example, 3000 seconds or shorter, 2500 seconds or shorter, 2000 seconds or shorter, or 1500 seconds or shorter from the viewpoint of shortening the reaction time.

The continuation time of the magnetic field exertion state, the continuation time of the no magnetic field state, the shaking or stirring rate, the number of cycles, the total reaction time, and the like can be changed as appropriate depending on the amount of the reaction solution, the kind of the substance of interest, the kind of the magnetic particles, the kind of the specific binding substance, or the like.

The composition of the reaction solution 40 is not particularly limited and can be selected as appropriate depending on the kinds of the substance of interest and the specific binding substance. As the reaction solution 40, for example, a reaction solution that is normally used in ELISA or the like can be used with no particular limitations. Examples of the reaction solution include buffers such as a phosphate buffer, PBS, a tris buffer, and an HEPES buffer, solutions obtained by adding a surfactant such as Tween20 to the above-described buffer, and the like.

The concentration of the magnetic particles 10 in the reaction solution 40 is not particularly limited and can be selected as appropriate depending on the kinds of the substance of interest and the specific binding substance. Examples of the concentration of the magnetic particles 10 in the reaction solution 40 include 1 to 500 ng/µL, 5 to 200 ng/µL, 10 to 100 ng/µL, 10 to 50 ng/µL, or the like.

The reaction temperature is not particularly limited and may be selected as appropriate depending on the kinds of the substance of interest and the specific binding substance. The reaction temperature is preferably 20°C or higher, preferably 30°C or higher, and more preferably 35°C or higher. The upper limit of the reaction temperature is, for example, preferably 50°C or lower, more preferably 45°C or lower, and still more preferably 40°C or lower. Examples of the range of the reaction temperature include 20°C to 50°C, 30°C to 45°C, 30°C to 40°C, or the like.

In the method of the present embodiment, the momentum of the magnetic particles in the reaction solution is increased by changing the magnetic field that is exerted on the magnetic particles in the binding reaction between the substance of interest and the magnetic particles. This increases the encounter probability of the substance of interest and the magnetic particles and accelerates the formation of the conjugates of the substance of interest and the magnetic particle. Therefore, it becomes possible to shorten the reaction time necessary for the formation of the conjugates.

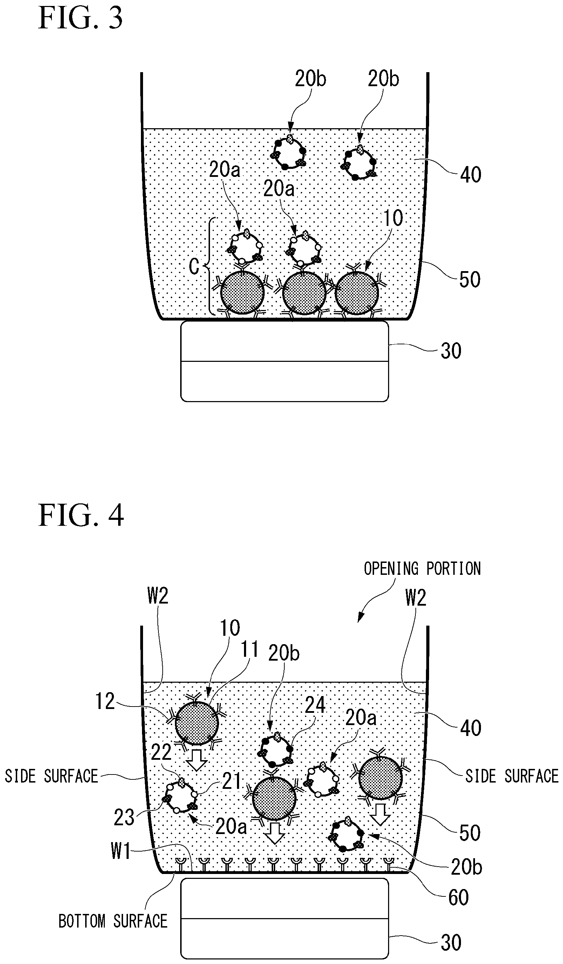

After the step (A1), the magnetic particles including the conjugates of the substance of interest and the magnetic particle may be collected with the magnet or the like, and the substance of interest is eluted from the magnetic particles and may be used for analysis or the like. For example, as shown in FIG. 3, only 20a having the exosome membrane protein 21 forms the conjugate C with the magnetic particle 10. Therefore, when the magnetic particles 10 are attracted to the bottom surface of the reaction container 50 with the magnet 30, and the reaction solution 40 is removed, it is possible to separate the exosomes 20a from the exosomes 20b that do not form any conjugates C. Alternatively, a labeling specific binding substance obtained by binding a labeling substance (an enzyme, a fluorescent dye, or the like) to the second specific binding substance that has an ability to specifically bind to the substance of interest may also be used. The labeling specific binding substance labels the substance of interest in the collected conjugates, and the quantity of the substance of interest may be determined by measuring the label signal.

Alternatively, the quantity of the substance of interest may be determined through the magnetic particles by determining the quantity of the magnetic particles that have formed the conjugates by a method to be described below.

### <Substance of interest measurement method (1)>

In one embodiment, the present invention provides a substance of interest measurement method. A method of the present embodiment includes a step (a1) of forming conjugates of a substance of interest and a magnetic particle and a step (b1) of determining the quantity of the substance of interest by determining the quantity of the magnetic particles that form the conjugates by the method of the above-described embodiment.

### (Step (a1))

The step (a1) is a step of forming conjugates of a substance of interest and a magnetic particle by the method of the above-described embodiment. The present step can be performed in the same manner as in the method described in the <conjugate formation method (1)> section.

### (Step (b1))

The step (b1) is a step of determining the quantity of the substance of interest by determining the quantity of the magnetic particles that form the conjugates.

A method for determining the quantity of the magnetic particles that form the conjugates is not particularly limited. For example, the conjugates may be collected using the second specific binding substance that has an ability to specifically bind to the substance of interest, and the quantity of the magnetic particles in the collected conjugates may be determined.

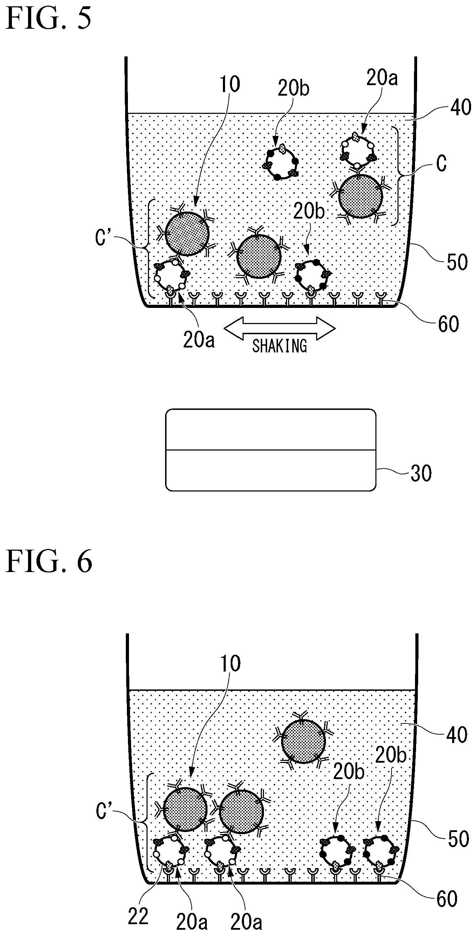

For example, it is possible to use the reaction container 50 in which a second specific binding substance 60 has been fixed to the bottom surface as shown in FIG. 6. The second specific binding substance 60 is, for example, an antibody that specifically binds to the exosome membrane protein 22 of the exosome 20a. When the second specific binding substance 60 has been fixed to the bottom surface of the reaction container 50, it is possible to bind the conjugates C to the bottom surface of the reaction container 50 through the binding between the exosome membrane protein 22 and the second specific binding substance 60. The magnetic particles 10 that do not form the conjugates C can be removed by removing the reaction solution 40 in this state.

The quantity of the magnetic particles that form the conjugates can be determined with, for example, a particle counter such as ExoCounter.

Alternatively, the quantity of the magnetic particles that form the conjugates can be determined by further binding a labeling substance to the magnetic particles and detecting the signal of the labeling substance. As the labeling substance, for example, a labeling substance that is normally used in ELISA or the like can be used with no particular limitations. Examples of the labeling substance include enzyme labels such as peroxidase (for example, horseradish peroxidase) and alkaline phosphatase; fluorescent labels such as carboxyfluorescein (FAM), 6-carboxy-4',5'-dichloro 2',7'-dimethoxyfluorescein (JOE), fluorescein isothiocyanate (FITC), tetrachlorofluorescein (TET), 5'-hexachloro-fluorescein-CE phosphoramidite (HEX), Cy3, Cy5, Alexa568, and Alexa647; radioisotope labels such as iodine-125; electrochemiluminescent labels such as ruthenium complex; biotin; and the like.

The signal of the labeling substance can be detected by a well-known method appropriate for the kind of the labeling substance. For example, in the case of an enzyme label, the signal of the labeling substance can be detected by performing a chromogenic reaction by an enzyme and detecting a chromogen. In addition, in a case where the labeling substance is a fluorescent dye, a radioisotope label, or an electrochemiluminescent label, the signal of the labeling substance can be detected by detecting a fluorescent signal, a radioisotopic signal, or an electrochemiluminescent signal.

In the case of using the reaction container 50 in which the second specific binding substance 60 has been fixed to the inner surface as shown in FIG. 6, the binding reaction between the second specific binding substance and the substance of interest may be performed at the same time as or separately from the step (a1), but is preferably performed at the same time in order to shorten the reaction time. In this case, as the reaction container that is used in the step (a1) (that is, the step (A1) in the method of the above-described embodiment), a reaction container in which the second specific binding substance has been fixed to the inner surface needs to be used. In the example of FIG. 6, the second specific binding substance is fixed to the bottom surface of the reaction container 50, but the fixation place is not limited to the bottom surface. The fixation place needs to be the inner surface of the reaction container 50 and may be, for example, an inner side surface or both the inner side surface and the bottom surface. When the step (a1) is performed using the reaction container to which the second specific binding substance has fixed, the binding reaction between the second specific binding substance and the substance of interest and the binding reaction between the specific binding substance bound to the magnetic particles and the substance of interest proceed at the same time. In this case, the magnet, which serves as a magnetic field generation source, is preferably disposed close to the inner surface to which the second specific binding substance has been fixed in the magnetic field exertion state. That is, in a case where the second specific binding substance has been fixed to the bottom surface of the reaction container, the magnet is preferably disposed on the bottom surface side of the reaction container.

In a case where the binding reaction between the second specific binding substance and the substance of interest is performed separately from the step (a1), first, it is possible to perform the binding reaction between the substance of interest and the second specific binding substance by adding a sample containing the substance of interest to the reaction container in which the second specific binding substance has been fixed to the inner surface. Next, the binding reaction between the first specific binding substance and the substance of interest can be performed by adding the magnetic particles to which the first specific binding substance has been fixed to the reaction container. In this case, it is preferable to remove the reaction solution and wash the reaction container before the addition of the magnetic particles to the reaction container. For the washing of the reaction solution, a washing solution that is used in ELISA or the like can be used with no particular limitations. Examples of the washing solution include buffers, solutions obtained by adding a surfactant such as Tween20 to the above-described buffer, and the like. Examples of the washing solution include buffers such as a phosphate buffer, PBS, a tris buffer, and an HEPES buffer, solutions obtained by adding a surfactant such as Tween20 to the above-described buffer, and the like, but the washing solution is not limited thereto.

### (Other steps)

The method of the present embodiment may include other steps in addition to the step (a1) and the step (b1). Examples of the other steps include a blocking step, a washing step, and the like.

### «Blocking step»

In the case of using the reaction container 50 in which the second specific binding substance 60 has been fixed to the inner surface as shown in FIG. 6, it is preferable to perform a blocking treatment of the reaction container 50 before the binding reaction between the second specific binding substance and the substance of interest is performed. Blocking makes it possible to suppress the non-specific binding to the inner wall of the reaction container 50.

The blocking treatment can be performed using a well-known blocking solution. As the blocking solution, for example, a blocking solution that is normally used in ELISA or the like can be used with no particular limitations. Examples of the blocking solution include buffers containing approximately 1% to 5% of skim milk or bovine serum albumin (BSA) and the like. Examples of the buffers as the blocking solution include buffers such as a phosphate buffer, PBS, a tris buffer, and an HEPES buffer, solutions obtained by adding a surfactant such as Tween20 to the above-described buffer, and the like, but the washing solution is not limited thereto.

### <<Washing step>>

After the step (a1) and before the step (b1), a washing treatment may be performed on the conjugates and/or the reaction container. The washing treatment can be performed by repeating a process of fixing the conjugates to the inner wall surface (preferably the bottom surface) of the reaction container as in FIG. 3 or 6, removing the reaction solution, and adding and removing the washing solution to and from the reaction container approximately once to three times. Examples of the washing solution include the same solutions as described above. The washing step removes substances that are not the substance of interest and do not bind to the magnetic particles from the reaction container and makes it possible to reduce measurement noise.

According to the method of the present embodiment, the binding reaction between the substance of interest and the magnetic particles is performed by the method of the above-described method, which makes it possible to reduce the time necessary for the binding reaction. Therefore, it is possible to reduce the time necessary for the measurement of the substance of interest and to rapidly determine the quantity of the substance of interest.

The method of the present embodiment can be used to determine the quantity of an arbitrary substance of interest in a sample. For example, the method of the present embodiment can be used to determine the quantities of exosomes, peptides, proteins, antibodies, sugar chains, lipids, nucleic acids, and the like that are contained in blood samples, cell culture media, cell lysates, and the like.

### <Kit (1)>

In one embodiment, the present invention provides a kit that is used in the methods of the embodiment. The kit of the present embodiment includes magnetic particles to which a specific binding substance that has an ability to specifically bind to a substance of interest has been bound, a reaction container in which the substance of interest and the magnetic particles are reacted, and a magnet. Alternatively, the kit of the present embodiment includes magnetic particles to which a specific binding substance that has an ability to specifically bind to a substance of interest can be bound, a reaction container in which the substance of interest and the magnetic particles are reacted, and a magnet.

### (Magnetic particles)

The kit of the present embodiment includes magnetic particles. In one embodiment, a specific binding substance that has an ability to specifically bind to a substance of interest has been bound to the magnetic particles. Examples of the magnetic particles include the same magnetic particles as those exemplified in the <conjugate formation method (1)> section.

The specific binding substance bound to the magnetic particles can be selected as appropriate depending on the substance of interest. Examples of the specific binding substance include the same specific binding substances as those exemplified in the conjugate formation method (1)> section. For example, in a case where the substance of interest is an exosome, examples of the specific binding substance include an antibody that specifically binds to an exosome membrane protein, a lectin that specifically binds to an exosome membrane sugar chain, or the like.

In another embodiment, the magnetic particles are magnetic particles to which the specific binding substance to the substance of interest can be bound. In this case, the specific binding substance is not yet bound to the magnetic particles, but the surfaces of the magnetic particles have been adapted to enable the specific substance to bind thereto at an arbitrary timing. For example, the magnetic particles may have particle surfaces to which the specific binding substance can be bound by physical adsorption or chemical bond. In the magnetic particles, the particle surfaces may be modified (for example, modified with an amino group, modified with a hydroxyl group, modified with a carboxy group, modified with a succinimide group, or the like) so as to have a specific functional group. The use of the specific binding substance having a functional group that can be bound to the above-described specific functional group makes it possible to bond the specific binding substance to the magnetic particles. Alternatively, the surfaces of the magnetic particles may be modified with biotin or modified with avidin. The use of a specific binding substance modified with avidin with respect to magnetic particles modified with biotin makes it possible to bond the specific binding substance to the magnetic particles. The use of a specific binding substance modified with biotin with respect to magnetic particles modified with avidin makes it possible to bond the specific binding substance to the magnetic particles. The avidin may be an avidin derivative such as streptavidin.

In a case where the kit of the present embodiment includes magnetic particles to which a specific binding substance to the substance of interest can be bound, a user who has purchased the kit is able to bind a desired specific binding substance to the magnetic particles.

### (Reaction container)

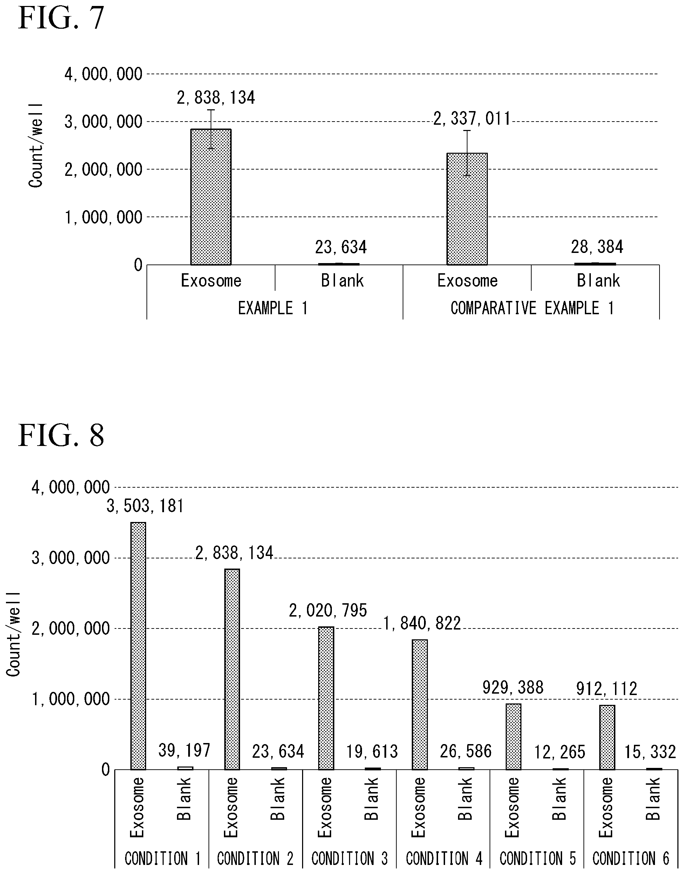

The reaction container is not particularly limited as long as the substance of interest and the magnetic particles can be reacted with each other. The reaction container is preferably formed of a material to which the non-specific adsorption of the substance of interest is less likely to occur. Examples of the material of the reaction container include resins such as polystyrenes, polyolefins (polyethylene, polypropylene, and the like), cycloolefin polymers, polycarbonates; glass; and the like, but the material is not limited thereto. The reaction container may be a well plate that is used in ELISA or the like. Alternatively, the reaction container may be a cartridge including wells attached to a particle counter such as ExoCounter or the like (refer to FIGS 7 and 8 of Japanese Unexamined Patent Application, First Publication No. 2017-040595).

In the reaction container, the second specific binding substance (capture specific binding substance) may have been fixed to the inner wall surface (for example, the bottom surface). The second specific binding substance may be a specific binding substance that has an ability to specifically bind to the substance of interest and binds to a portion different from the first specific binding substance (for example, a different epitope, a different membrane protein, or the like).

### (Magnet)

The magnet may be a permanent magnet or may be an electromagnet. In the case of an electromagnet, the magnet may be integrated with the wall surface of the reaction container. The size of the magnet is not particularly limited and can be selected as appropriate depending on the size of the reaction container. The magnetic force of the magnet is not particularly limited and can be selected as appropriate depending on the size of the reaction container, the kind of the magnetic particles, or the like. Examples of the magnetic force of the magnet include magnetic flux densities of 0.1 to 0.6 tesla.

A magnet built in a shaker or the like may be used as the magnet. The shaker may be, for example, a shaker that can be set to alternately repeat the attraction of the magnetic particles with the built-in magnet and shaking at arbitrary intervals.

### (Arbitrary configuration)

A measurement kit of the present embodiment may include an arbitrary configuration in addition to the above-described configuration. Examples of the arbitrary configuration include a reagent for treating a sample (for example, a diluted solution or the like), a variety of reagents such as a washing solution, a blocking solution, and a buffer, instructions for use, and the like.

### <Conjugate formation method (2)>

In one embodiment, the present invention provides a method for forming a conjugate of a substance of interest, a magnetic particle to which a first specific binding substance that has an ability to specifically bind to the substance of interest has been bound, and a second specific binding substance that has an ability to specifically bind to the substance of interest and has been bound to an inner wall of a reaction container. The method of the present embodiment includes a step (A2) of performing a binding reaction between the substance of interest and the magnetic particle and a binding reaction between the substance of interest and the second specific binding substance in a reaction solution accommodated in the reaction container and containing the substance of interest and the magnetic particles. The step (A2) includes increasing the momentum of the magnetic particles in the reaction solution by changing the magnetic field that is exerted on the magnetic particles.

Examples of the substance of interest include the same substances of interest as those exemplified in the <conjugate formation method (1)> section.

The substance of interest may be, for example, a substance that is contained in a biological sample or the like. Examples of the biological sample include the same biological samples as those exemplified in the <conjugate formation method (1)> section.

The first specific binding substance and the second specific binding substance can be selected as appropriate depending on the substance of interest. For example, in a case where the substance of interest is an exosome, examples of the specific binding substance include an antibody that specifically binds to a membrane protein of the exosome, a lectin that specifically binds to a sugar chain of the exosome, and the like. The membrane protein of sugar chain of the exosome may be a membrane protein of sugar chain that is widely expressed in exosomes or may be a membrane protein or sugar chain that is expressed only in an exosome discharged from a specific cell (for example, a cancer cell). Examples of the membrane protein that is widely expressed in exosomes (hereinafter, also referred to as "wide exosome membrane protein") include CD9, CD63, CD81, and the like.

The first specific binding substance and the second specific binding substance may be the same as or different from each other. For example, in a case where the substance of interest is an exosome, the first specific binding substance and the second specific binding substance may be each an antibody that specifically binds to a different exosome membrane protein. Alternatively, in a case where the substance of interest is an exosome, the first specific binding substance and the second specific binding substance may be antibodies that specifically bind to the same exosome membrane protein. Normally, an exosome expresses a plurality of the same exosome membrane proteins. Therefore, in a case where the first specific binding substance and the second specific binding substance are antibodies that specifically bind to the same exosome membrane protein, the first specific binding substance and the second specific binding substance may be substances that recognize the same epitope of the exosome membrane protein or may be each a substance that recognizes a different epitope. For example, in a case where the substance of interest is a peptide or a protein, the first specific binding substance and the second specific binding substance are each preferably a substance that recognizes a different epitope.

As the magnetic particles, the same magnetic particles as those exemplified in the <conjugate formation method (1)> section can be used.

In the method of the present embodiment, magnetic particles to which the first specific binding substance has been bound are used as the magnetic particles. Examples of a method for binding the first specific binding substance and the magnetic particle include the methods exemplified in the <conjugate formation method (1)> section.

"Reaction container" is a component for accommodating the reaction solution containing the substance of interest and the magnetic particles. The reaction container is not particularly limited as long as the reaction solution can be accommodated. The reaction container is preferably formed of a material to which the non-specific adsorption of the substance of interest is less likely to occur. Examples of the material of the reaction container include the same materials as those exemplified in the <kit (1)> section.

In the method of the present embodiment, a reaction container to which the second specific binding substance has been bound to the inner wall is used as the reaction container. The second specific binding substance can be bound to the inner wall of the reaction container by a well-known method depending on the kind of the second specific binding substance. Examples of a method for binding the second specific binding substance to the inner wall of the reaction container include the same methods as those exemplified as the method for binding the first specific binding substance to the magnetic particles.

The inner wall of the reaction container to which the second specific binding substance is bound may be the inner wall of the side surface (for example, an inner wall W2 in FIG. 4) of the reaction container or may be the inner wall of the bottom surface (for example, an inner wall W1 in FIG. 4). From the viewpoint of the stability of the conjugates, the second specific binding substance is preferably bound to the inner wall of the bottom surface of the reaction container. The second specific binding substance may be bound to both the inner wall of the bottom surface and the inner wall of the side surface of the reaction container. The second specific binding substance may be bound to only a part of the inner wall of the reaction container or may be bound to the entire surface of the inner wall of the reaction container.

### (Step (A2))

The step (A2) is a step of performing a binding reaction between the substance of interest and the magnetic particle and a binding reaction between the substance of interest and the second specific binding substance in the reaction solution accommodated in the reaction container and containing the substance of interest and the magnetic particles. The step (A2) includes increasing the momentum of the magnetic particles in the reaction solution by changing the magnetic field that is exerted on the magnetic particles.

FIG. 4 is a view for schematically describing an example of the step (A2). In an example of FIG. 4, a case where the substance of interest is an exosome is shown. In FIG. 4, a reaction solution 40 is accommodated in a reaction container 50 in which a second specific binding substance 60 has been bound to the inner wall W1 of the bottom surface. The reaction solution 40 contains exosomes 20a, exosomes 20b, and magnetic particles 11 to which a first specific binding substance 12 has been bound (magnetic particles 10). The exosome 20a contains exosome membrane proteins 21, 22 and 23. The exosome 20b contains exosome membrane proteins 22, 23 and 24. The first specific binding substance 12 is, for example, an antibody that specifically binds to the exosome membrane protein 21. Therefore, the magnetic particles 10 bind to the exosomes 20a, but do not bind to the exosomes 20b. The second specific binding substance 60 is, for example, an antibody that specifically binds to the exosome membrane protein 22. The exosomes 20a and 20b both contain the exosome membrane protein 22. Therefore, the exosomes 20a and 20b are both captured by the second specific binding substance 60.

In the reaction solution containing the substance of interest and the magnetic particles, a change in the magnetic field that is exerted on the magnetic particles increases the momentum of the magnetic particles due to the exertion of the magnetic field. "A change in the magnetic field that is exerted on the magnetic particles 10" may be a change in the intensity of the magnetic field that is exerted on the magnetic particles or may be a change in the direction of the magnetic field that is exerted on the magnetic particles. "A change in the magnetic field that is exerted on the magnetic particles 10" is preferably a change in the intensity of the magnetic field that is exerted on the magnetic particles. For example, in the example of FIG. 4, when a magnet 30 comes close to the bottom surface of the reaction container 50, the magnetic field that is exerted on the magnetic particles 10 becomes strong. This makes the magnetic particles 10 attracted in a direction toward the magnet 30, which is the generation source of the magnetic field, and thus makes it possible to increase the momentum of the magnetic particles 10.

The increase in the momentum of the magnetic particles 10 increase the encounter probability of the magnetic particle 10 and the exosome 20. This makes it possible to accelerate the binding reaction between the exosome 20a and the magnetic particle 10 (more specifically, the binding reaction between the exosome membrane protein 21 and the first specific binding substance 12). Therefore, the formation of conjugates C of the exosome 20a and the magnetic particle 10 is accelerated (refer to FIG. 5).

The magnet 30 may be brought close in the side surface direction of the reaction container 50. For example, in a case where there are no other reaction containers in the side surface direction of the reaction container 50, the magnet 30 can be brought close in the side surface direction of the reaction container 50. In addition, the magnet 30 may be brought close in the opening portion direction of the reaction container 50.

Alternatively, the position of the magnet 30 may be sequentially switched between any two or more positions selected in the bottom surface direction, the side surface direction, and the opening portion direction. A change in the position of the magnet 30 makes it possible to change the direction of the magnetic field that is exerted on the magnetic particles 10. Since the magnetic particles 10 are attracted in a direction toward the magnet 30 depending on the change in the position of the magnet 30, the momentum of the magnetic particles 10 can be increased.

The magnet 30 is preferably brought close to the reaction container 50 in a direction toward the inner wall to which the second specific binding substance 60 has been bound. That is, the magnet 30 is preferably disposed close to the inner wall to which the second specific binding substance 60 has been bound. This makes it possible to attract the conjugates C of the exosome 20a and the magnetic particle 10 in a direction toward the inner wall to which the second specific binding substance 60 has been bound. As a result, the encounter probability of the exosome 20a configuring the conjugate C and the second specific binding substance 60 becomes high, and it is possible to accelerate the binding reaction between the exosome 20a configuring the conjugate C and the second specific binding substance 60.

In a case where the second specific binding substance 60 has been bound to the inner wall W1 of the bottom surface of the reaction container 50, the magnet 30 is preferably brought close to the inner wall W1 of the bottom surface of the reaction container 50. This makes the magnetic particles 10 attracted in a direction toward the bottom surface of the reaction container 50 and increases the momentum of the magnetic particles 10. As a result, it is possible to accelerated both the binding reaction between the exosome 20a and the magnetic particle 10 and the binding reaction between the exosome 20a configuring the conjugate C and the second specific binding substance 60. Therefore, it is possible to accelerate the formation of conjugates C' of the exosome 20a, the magnetic particle 10 and the second specific binding substance 60 (refer to FIGS. 4 and 5).

The magnet 30 may be a permanent magnet or may be an electromagnet. In a case where the magnet 30 is an electromagnet, the magnetic field can be changed by changing the amount of electricity conducted. Therefore, it is not necessary to move the magnet 30 with respect to the reaction container 50.

In a case where the magnet 30 is a permanent magnet, the magnet 30 is preferably present outside the reaction container 50. In a case where the magnet 30 is an electromagnet, the magnet 30 may be present outside the reaction container 50 or may be integrated with the bottom surface or side surface of the reaction container 50.

In the step (A2), after the magnetic field that is exerted on the magnetic particles 10 is changed, it is preferable to maintain the state after the change for a certain period of time. It is preferable to alternately repeat the magnetic field exertion state and the no magnetic field state. From the viewpoint of improvement in the formation efficiency of the conjugates C' and reduction in non-specific binding, the continuation time of the magnetic field exertion state is preferably 30 seconds or shorter, more preferably 20 seconds or shorter, still more preferably 10 seconds or shorter, and particularly preferably six seconds or shorter or three seconds or shorter. The lower limit of the continuation time of the magnetic field exertion state is not particularly limited as long as the momentum of the magnetic particles 10 can be increased during that time. Examples of the lower limit of the continuation time of the magnetic field exertion state include 0.5 seconds or longer, one second or longer, 1.2 seconds or longer, or 1.5 seconds or longer.

In a case where the magnet 30 is a permanent magnet, the magnetic field exertion state may be continued by continuing a state where the magnet 30 has been brought close to the reaction container 50 (the bottom surface, side surface or opening portion of the reaction container 50). The magnetic field exertion state can be continued by maintaining a state where the magnet 30 has been brought close to the outer wall corresponding to the inner wall of the reaction container 50 to which the second specific binding substance 60 has been bound. In a case where the magnet 30 is an electromagnet, the magnetic field exertion state may be continued by continuing a state where the electricity has been conducted in the magnet 30.

The continuation time of the no magnetic field state is not particularly limited; however, from the viewpoint of accelerating the formation of the conjugates C', the continuation time can be set to, for example, 60 seconds or shorter and is preferably 40 seconds or shorter, more preferably magnet 30 seconds or shorter, still more preferably 20 seconds or shorter, and particularly preferably 15 seconds or shorter or 10 seconds or shorter. The lower limit of the continuation time of the no magnetic field state is not particularly limited, and, from the viewpoint of reducing non-specific binding, examples thereof include 0.5 seconds or longer, one second or longer, two seconds or longer, three seconds or longer, or five seconds or longer.

In a case where the magnet 30 is a permanent magnet, the no magnetic field state may be continued by continuing a state where the magnet 30 has been separated from the reaction container 50 (the bottom surface, side surface, or opening portion of the reaction container 50) by a distance at which the magnetic field attributed to the magnet 30 is not exerted. In a case where the magnet 30 is an electromagnet, the no magnetic field state may be continued by continuing a state where the electricity is not conducted in the magnet 30.

The intensity of the magnetic force of the magnet 30 is not particularly limited as long as the momentum of the magnetic particles 10 can be increased. Examples of the intensity of the magnetic force of the magnet 30 include magnetic flux densities of 0.1 to 0.6 tesla.

The step (A2) may further include stirring or shaking the reaction solution containing the substance of interest and the magnetic particles (FIG. 5). The stirring or shaking makes it possible to improve the formation efficiency of the conjugates C. The stirring or shaking can be performed using, for example, a shaker or the like When the reaction solution is shaken or stirred in the magnetic field exertion state, the reaction solution 40 swirls, and there are cases where the magnetic particles 10 are not evenly distributed. Therefore, the reaction solution is preferably shaken or stirred in the no magnetic field state as shown in FIG. 5.

The shaking or stirring rate is not particularly limited and can be, for example, 100 to 3000 rpm.

Examples of the number of cycles of the magnetic field exertion state and the no magnetic field state include the same numbers of cycles as those exemplified in the

### <conjugate formation method (1)> section.

Any of the magnetic field exertion state and the no magnetic field state may be performed earlier. For example, at the time of beginning the step (A2), the step may begin in the magnetic field exertion state, next, the no magnetic field state may follow, and then, the magnetic field exertion state and the no magnetic field state may be alternately repeated. Alternatively, at the time of beginning the step (A2), the step may begin in the no magnetic field state, next, the magnetic field exertion state may follow, and then, the no magnetic field state and the magnetic field exertion state may be alternately repeated.

Examples of the total reaction time including the reaction time in the magnetic field exertion state and the reaction time in the no magnetic field state include the same total reaction times as those exemplified in the <conjugate formation method (1)> section.

The continuation time of the magnetic field exertion state, the continuation time of the no magnetic field state, the shaking or stirring rate, the number of cycles, the total reaction time, and the like can be changed as appropriate depending on the amount of the reaction solution, the kind of the substance of interest, the kind of the magnetic particles, the kind of the specific binding substance, or the like.

Examples of the composition of the reaction solution 40, the concentration of the magnetic particles 10 in the reaction solution 40, and the reaction temperature include the same compositions, concentrations, and reaction temperatures as those exemplified in the

### <conjugate formation method (1)> section.

FIG. 6 is a view for schematically describing an example of a state of the reaction container after the step (A2). The step (A2) forms the conjugates C' of the exosome 20a, the magnetic particle 10 and the second specific binding substance 60. The conjugates C' are fixed to the inner wall of the reaction container 50 through the second specific binding substance 60. Incidentally, in the reaction solution 40 after the step (A2), in addition to the conjugates C', the unreacted magnetic particles 10 that do not form any conjugates, the exosomes 20b to which the magnetic particle 10 does not bind, and the like are present. The unreacted magnetic particles 10 can be removed from the reaction container 50 by removing the reaction solution 40.

In the method of the present embodiment, the momentum of the magnetic particles in the reaction solution is increased by changing the magnetic field that is exerted on the magnetic particles in the reaction solution containing the substance of interest and the magnetic particles. This increases the encounter probability of the substance of interest and the magnetic particles and accelerates the formation of the conjugates of the substance of interest and the magnetic particle. Furthermore, since the momentum of the conjugates of the substance of interest and the magnetic particle also increases, the encounter probability of the substance of interest configuring the conjugates and the second specific binding substance bound to the inner wall of the reaction container also becomes high. This also accelerates the formation of the conjugates of the substance of interest, the magnetic particle, and the second specific binding substance. Therefore, it becomes possible to shorten the reaction time necessary for the formation of the conjugates.

After the step (A2), the quantity of the magnetic particles that form the conjugates of the substance of interest, the magnetic particle, and the second specific binding substance is determined by a method to be described below, whereby the quantity of the substance of interest can be determined through the magnetic particles.

### <Substance of interest measurement method (2)>

In one embodiment, the present invention provides a substance of interest measurement method. A method of the present embodiment includes a step (a2) of forming conjugates of a substance of interest, a magnetic particle, and a second specific binding substance and a step (b2) of determining the quantity of the substance of interest by determining the quantity of the magnetic particles that form the conjugates.

### (Step (a2))

The step (a2) is a step of forming conjugates of a substance of interest, a magnetic particle, and a second specific binding substance by the method of the above-described embodiment. The present step can be performed in the same manner as in the method described in the <conjugate formation method (2)> section.

### (Step (b2))

The step (b2) is a step of determining the quantity of the substance of interest by determining the quantity of the magnetic particles that form the conjugates.

A method for determining the quantity of the magnetic particles that form the conjugates is not particularly limited. The reaction container after the step (a2) is in a state where the conjugates C' of the substance of interest, the magnetic particle, and the second specific binding substance have been formed and the conjugates C' have been captured on the inner wall of the reaction container 50 through the second specific binding substance as shown in FIG. 6. The magnetic particles 10 that do not form the conjugates C' can be removed by removing the reaction solution 40 in this state.

The quantity of the magnetic particles that form the conjugates C' can be determined with, for example, a particle counter such as ExoCounter. For example, when a well provided on a disc or the like attached to a particle counter or the like is used as the reaction container, it becomes easy to determine the quantity of the magnetic particles in the particle counter.

Alternatively, the quantity of the magnetic particles that form the conjugates can be determined by further binding a labeling substance to the magnetic particles and detecting the signal of the labeling substance. Examples of the labeling substance include the same labeling substances as those exemplified in the <substance of interest measurement method (1)> section. Examples of a method for detecting the signal of the labeling substance include the same methods as the methods exemplified in the

### <substance of interest measurement method (1)> section.

### (Other steps)

The method of the present embodiment may include other steps in addition to the step (a2) and the step (b2). Examples of the other steps include a blocking step, a washing step, and the like.

### «Blocking step»

A blocking treatment makes it possible to suppress the non-specific binding to the inner wall of the reaction container. The blocking step can be performed in the same manner as in the method exemplified in the <substance of interest measurement method (1)> section.

### <<Washing step>>

After the step (a2) and before the step (b2), a washing treatment may be performed on the conjugates and the reaction container. The washing treatment can be performed by repeating a process of removing the reaction solution and adding and removing the washing solution to and from the reaction container approximately once to three times after the step (a2). Examples of the washing solution include the same solutions as described above. The washing step removes contaminants contained in the reaction solution from the reaction container and makes it possible to reduce measurement noise.

According to the method of the present embodiment, the binding reaction in the step (a1) is performed by the method of the above-described method, which makes it possible to reduce the time necessary for the binding reaction. Therefore, it is possible to reduce the time necessary for the measurement of the substance of interest and to rapidly determine the quantity of the substance of interest.

The method of the present embodiment can be used to determine the quantity of an arbitrary substance of interest in a sample. For example, the method of the present embodiment can be used to determine the quantities of exosomes, peptides, proteins, antibodies, sugar chains, lipids, nucleic acids, and the like that are contained in blood samples, cell culture media, cell lysates, and the like.

### <Kit (2)>

In one embodiment, the present invention provides a kit that is used in the methods of the present embodiment. The kit of the present embodiment includes magnetic particles to which a first specific binding substance to a substance of interest has been bound or magnetic particles to which a first specific binding substance to a substance of interest can be bound, a reaction container in which a second specific binding substance to the substance of interest has been bound to the inner wall or a reaction container including an inner wall to which a second specific binding substance to the substance of interest can be bound, and a magnet.

### (Magnetic particles)

The kit of the present embodiment includes magnetic particles. In one embodiment, a first specific binding substance that has an ability to specifically bind to a substance of interest has been bound to the magnetic particles. Examples of the magnetic particles include the same magnetic particles as those exemplified in the

### <conjugate formation method (1)> section.

The first specific binding substance bound to the magnetic particles can be selected as appropriate depending on the substance of interest. Examples of the first specific binding substance include the same first specific binding substances as those exemplified in the <conjugate formation method (2)> section. For example, in a case where the substance of interest is an exosome, examples of the first specific binding substance include an antibody that specifically binds to an exosome membrane protein, a lectin that specifically binds to an exosome membrane sugar chain, and the like.

In another embodiment, the magnetic particles are magnetic particles to which the first specific binding substance to the substance of interest can be bound. In this case, the first specific binding substance is not yet bound to the magnetic particles, but the surfaces of the magnetic particles have been adapted to enable the first specific binding substance to bind thereto at an arbitrary timing. Examples of the magnetic particles in this case include the same magnetic particles as those exemplified in the <kit (1)>.

In a case where the kit of the present embodiment include magnetic particles to which the first specific binding substance that has an ability to specifically bind to the substance of interest can be bound, a user who has purchased the kit is able of bind a desired first specific binding substance to the magnetic particles.

### (Reaction container)

The kit of the present embodiment includes a reaction container. In one embodiment, a second specific binding substance that has an ability to specifically bind to the substance of interest has been bound to the inner wall of the reaction container. Examples of the reaction container include the same reaction containers as those exemplified in the <conjugate formation method (2)> section. The inner wall to which the second specific binding substance has been bound may be any of the side surface and bottom surface of the reaction container, but is preferably the inner wall of the bottom surface. The second specific binding substance may be bound to a part of the inner wall or may be bound to the entire surface of the inner wall.

The second specific binding substance bound to the inner wall of the reaction container can be selected as appropriate depending on the substance of interest. Examples of the second specific binding substance include the same second specific binding substances as those exemplified in the <conjugate formation method (2)> section. For example, in a case where the substance of interest is an exosome, examples of the second specific binding substance include an antibody that specifically binds to an exosome membrane protein, a lectin that specifically binds to an exosome membrane sugar chain, and the like. The second specific binding substance and the first specific binding substance may be the same as or different from each other.

In another embodiment, the reaction container includes an inner wall to which the second specific binding substance to the substance of interest can be bound. In this case, the specific binding substance is not yet bound to the inner wall of the reaction container, but the inner wall of the reaction container has been adapted to enable the second specific binding substance to bind thereto at an arbitrary timing. For example, the reaction container may have an inner wall to which the second specific binding substance can be bound by physical adsorption or chemical bond. The inner wall of the reaction container may be modified (for example, modified with an amino group, modified with a hydroxyl group, modified with a carboxy group, modified with a succinimide group, or the like) so as to have a specific functional group. The use of the second specific binding substance having a functional group that can be bound to the above-described specific functional group makes it possible to bond the second specific binding substance to the inner wall of the reaction container. Alternatively, the reaction container may include an inner wall modified with biotin or modified with avidin. The use of the second specific binding substance modified with avidin with respect to the inner wall modified with biotin makes it possible to bond the second specific binding substance to the inner wall. The use of the second specific binding substance modified with biotin with respect to the inner wall modified with avidin makes it possible to bond the second specific binding substance to the inner wall. The avidin may be an avidin derivative such as streptavidin.

The inner wall to which the second specific binding substance can be bound may be provided on the bottom surface of the reaction container or may be provided on the side surface, but is preferably provided on the bottom surface. A region in the inner wall of the reaction container to which the second specific binding substance can be bound may be a part of the inner wall or may be the entire surface.

The material of the reaction container is not particularly limited. Examples of the material of the reaction container include the same materials as those exemplified in the <kit (1)>.

### (Magnet)

The magnet may be a permanent magnet or may be an electromagnet. Examples of the magnet include the same magnets as those exemplified in the <kit (1)>.

### (Arbitrary configuration)

A measurement kit of the present embodiment may include an arbitrary configuration in addition to the above-described configuration. Examples of the arbitrary configuration include a reagent for treating a sample (for example, a diluted solution or the like), a variety of reagents such as a washing solution, a blocking solution, and a buffer, instructions for use, and the like.

### [Examples]

Hereinafter, the present invention will be described with examples, but the present invention is not limited to the following examples.

In the following examples, examples where a counting system in which an exosome was used as a substance of interest, an antibody was used as a specific binding substance, and a laser optical pick-up was used for a method for measuring magnetic particles was used will be described, but the present invention is also applicable to sandwich immunassays and the like. As a reaction container, a well provided in a cartridge attached to ExoCounter (registered trademark) (JVCKENWOOD Corporation) was used.

### [Example 1]

### (Preparation of substance of interest)

As a substance of interest, an exosome extracted from a culture medium of a colon cancer cell line HCT116 was used. The extracted exosome was suspended in phosphate-buffered saline and used as an exosome-containing sample. A phosphate buffer containing no exosomes was prepared as an exosome-free sample.

### (Preparation of capture antibody)

An exosome monoclonal antibody Anti-CD9 (Cosmo Bio) was used as a capture antibody (second specific binding substance). 70 µL of a 5 µg/mL capture antibody solution was added dropwise to the well and then incubated at 37°C for 30 minutes to fix the capture antibody to the well. After the fixation of the capture antibody, the well was washed with a Tween20-containing phosphate buffer.

### (Blocking of well)

200 µL of a Tween20-containing phosphate buffer containing 1% BAS was added dropwise to the well to which the capture antibody had been fixed as described above and incubated at 37°C for 30 minutes, thereby performing the blocking of the well. After the blocking, the well was washed with a Tween20-containing phosphate buffer.

### (Mixing of exosome-containing sample and magnetic particles)

An Anti-CD9 antibody (Cosmo Bio) was used as a first specific binding substance. CD9 is known as a common marker for exosomes. FGBeads (registered trademark) (Tamagawa Seiki Co., Ltd.) to which the Anti-CD9 antibody had been fixed was used as magnetic particles. 25 µL of the exosome-containing sample or 25 µL of the exosome-free sample and 25 µL of the Tween20-containing phosphate buffer containing 1 µg of the magnetic particles were added dropwise to the well. The exosome-containing sample or the exosome-free sample and the magnetic particles were mixed in a separate container such as a 1.5 mL tube, and then the liquid mixture may be added dropwise to the well.

### (Reaction between exosome and magnetic particles)

A magnet (neodymium magnet (cylindrical, φ6 × 12), magnetic flux density: 0.52 Tesla) was brought close to the well bottom surface from the outside of the well, and the magnet was left still at the position where the magnet was in contact with the well bottom surface for 1.5 seconds (hereinafter, referred to as "magnetic sweep"). Next, the magnet was brought away from the well bottom surface, and the well was shaken and stirred (at 1200 rpm) for five seconds with a microplate mixer (AS ONE Corporation). The shaking and stirring after the magnetic sweep as described above was regarded as one cycle, and the process was performed 100 cycles. After that, the well was washed with a Tween20-containing phosphate buffer. For the magnetic sweep, a neodymium magnet having a size corresponding to the well was used. A device including a magnetic sweep system externally attached to a microplate mixer was self-made, and the magnetic sweep and the shaking and stirring were performed by automatic control.

### (Measurement)

The well was dried and then separated from the cartridge, and a disc for sample analysis was measured with ExoCounter. The numbers of the magnetic particles bound to the disc surfaces were counted in the well to which the exosome-containing sample had been added (Exosome) and the well to which the exosome-free sample had been added (Blank).

### [Comparative Example 1]

### (Preparation of substance of interest, preparation of capture antibody, and blocking of well)

The preparation of a substance of interest, the preparation of a capture antibody, and the blocking of a well were performed by the same methods as in Example 1.

### (Reaction between exosome and capture antibody)

50 µL of the exosome-containing sample or 50 µL of the exosome-free sample was added dropwise to the well and incubated at 37°C for 120 minutes. After the incubation, the well was washed with a Tween20-containing phosphate buffer.

### (Reaction between exosome and magnetic particles)

As a first specific binding substance and magnetic particles, the same first specific binding substance and magnetic particles as in Example 1 were used. 50 µL of a Tween20-containing phosphate buffer containing 1 µg of the magnetic particles was added to the well and incubated at 37°C for 90 minutes. After the incubation, the well was washed with a Tween20-containing phosphate buffer.

### (Measurement)

The preparation of a substance of interest, the preparation of a capture antibody, and the blocking of a well were performed by the same methods as in Example 1.

### (Results)

The measurement results of Example 1 and Comparative Example 1 are shown in FIG. 7. In Example 1, the counter value of the well to which the exosome-containing sample was added was 2,838,134. On the other hand, in Comparative Example 1, the counter value of the well to which the exosome-containing sample was added was 2,337,011. In Example 1, the counter value of the well to which the exosome-free sample was added was 23,634. On the other hand, in Comparative Example 1, the counter value of the well to which the exosome-free sample was added was 28,384. From these results, it was confirmed that, in Example 1, compared with Comparative Example 1, the count value with respect to the exosome-containing sample was high and the count value with respect to the exosome-free sample was low. In Comparative Example 1, 120 minutes was required for the reaction between the exosome and the capture antibody, 90 minutes was required for the reaction between the exosome and the magnetic particle, and a total of 210 minutes of a reaction time was required. On the other hand, in Example 1, the reaction time was approximately 10 minutes, and it was possible to precisely measure the exosome. From these results, it was confirmed that the use of the method of Example 1 makes it possible to significantly shorten the reaction time.

### [Example 2]

Steps were performed by the same methods as in Example 1 except that, in the reaction between an exosome and a magnetic particle, the magnetic sweep time, the shaking and stirring time, and the number of cycles were set to each condition shown in Table 1.

**[Table 1]**

| | Magnetic sweep (seconds) | Shaking and stirring (seconds) | Number of cycles (cycles) | Total reaction time (seconds) |
|---|---|---|---|---|
| Condition 1 | 1.5 | 10 | 100 | 1150 |
| Condition 2 | 1.5 | 5 | 100 | 650 |
| Condition 3 | 3 | 10 | 50 | 650 |
| Condition 4 | 3 | 5 | 50 | 400 |
| Condition 5 | 6 | 10 | 25 | 400 |
| Condition 6 | 10 | 10 | 15 | 300 |

### (Results)

The measurement result of Example 2 is shown in FIG. 8. The count values of the wells to which the exosome-containing sample had been added were increased by shortening the magnetic sweep time per cycle and increasing the number of cycles under conditions 1 to 6. From these results, it was confirmed that a larger number of conjugates of the exosome, the magnetic particle, and the capture antibody were formed by shortening the magnetic sweep time per cycle and increasing the number of cycles. In the wells to which the exosome-free sample had been added, no specific tendencies depending on the conditions 1 to 6 were shown. It is considered that, in order to satisfy both the improvement in the conjugate formation rate and the reduction in the nonspecific adsorption rate, it is desirable to adjust the magnetic sweep time, the shaking and stirring time, and the number of cycles in accordance with the objective of an experiment, a substance of interest, and the characteristics of an antibody used.

### [Example 3]

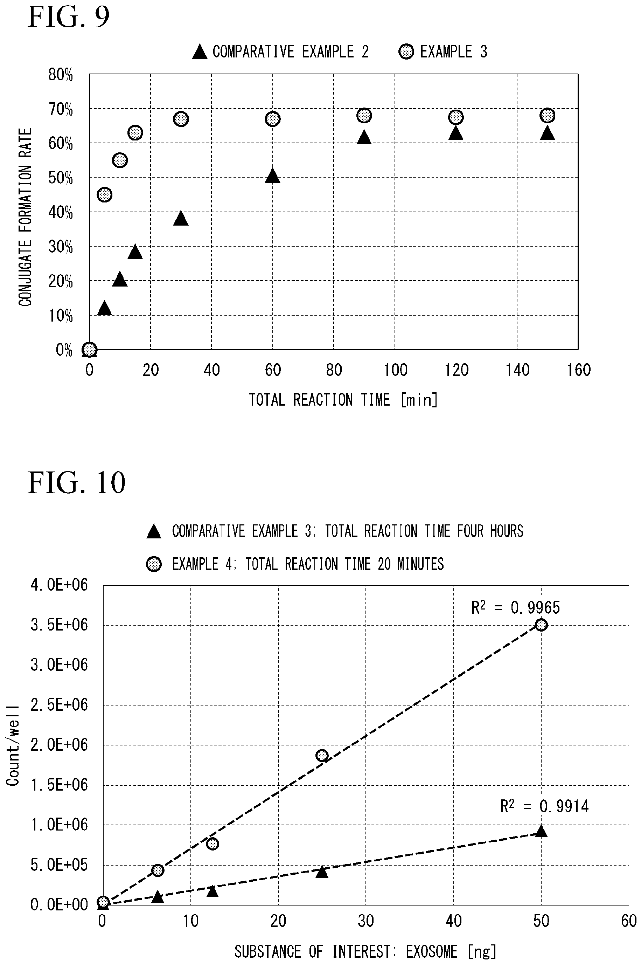

A reaction between an exosome and a magnetic particle was performed by the same method as in Example 1 except that a capture antibody was not fixed to a well, and, in the reaction between the exosome and the magnetic particle, the magnetic sweep time was set to three seconds, the shaking and stirring time was set to three seconds, and the number of cycles was changed to adjust the total reaction time to each total reaction time shown in FIG. 9.

### (Measurement)

A reaction solution was collected from the well in a state where the magnetic particles were captured on the bottom surface of the well using a magnet. Exosomes contained in the collected reaction solution were measured, and the quantity of the unreacted substance of interest not bound to the magnetic particles was determined. The conjugate formation rate was calculated from the amount of the unreacted substance of interest.

### [Comparative Example 2]

Steps were performed by the same methods as in Example 3 except that, in the reaction between an exosome and a magnetic particle, the magnetic sweep was not performed.

### (Results)

The results of Example 3 and Comparative Example 2 are shown in FIG. 9. It was confirmed that, in Example 3, compared with Comparative Example 2, the detection rate was saturated within a short amount of time.

### [Example 4]

Steps were performed by the same methods as in Example 1 except that the amount of an exosome in an exosome-containing sample was set to each amount shown in FIG. 10, and, in the reaction between the exosome and the magnetic particle, the magnetic sweep time was set to 1.5 seconds, the shaking and stirring time was set to 10 seconds, and the number of cycles was set to 100 times to make the total reaction time be 20 minutes.

### [Comparative Example 3]

Steps were performed by the same methods as in Comparative Example 1 except that the amount of an exosome in an exosome-containing sample was set to each amount shown in FIG. 10, and the total reaction time of the reaction time between the exosome and a capture antibody and the reaction time between the exosome and a magnetic particle was made to be four hours.

### (Results)

The results of Example 4 and Comparative Example 3 are shown in FIG. 10. It was confirmed that, in Example 4, compared with Comparative Example 3, the conjugate formation rate improved.

### [Industrial Applicability]

According to the present invention, a formation method of a conjugate of a substance of interest and a magnetic particle in which the reaction time between the substance of interest and the magnetic particle can be reduced, a substance of interest measurement method, and a kit that is used in the methods are provided.

In addition, according to the present invention, a conjugate formation method in which the reaction time among a substance of interest, a magnetic particle to which a first specific binding substance has been bound, and a second specific binding substance bound to a reaction container can be reduced, a substance of interest measurement method, and a kit that is used in the methods are provided.

Hitherto, the preferable embodiment of the present invention has been described and shown in the drawings, but this is an example of the present invention, and it should be understood that the present invention should not be regarded as being limited by the embodiment. Addition, omission, substitution, and other modifications can be performed within the sprit or scope of the present invention. Therefore, the present invention is not regarded as being limited by the above description and is limited only by the appended claims.

### [Reference Signs List]

10 Magnetic particle to which specific binding substance is bound
11 Magnetic particle
12 Specific binding substance
20a, 20b Exosome
21, 22, 23, 24 Exosome membrane protein
30 Magnet
40 Reaction solution
50 Reaction container
60 Second specific binding substance
C Conjugate of magnetic particle and exosome
C' Conjugate of magnetic particle, exosome, and second specific binding substance

## Claims

1. A method for forming a conjugate of a substance of interest and a magnetic particle to which a specific binding substance that has an ability to specifically bind to the substance of interest has been bound, the method comprising:
a step (A1) of reacting the substance of interest and the magnetic particles in a reaction solution,
wherein the step (A1) comprises increasing momentum of the magnetic particles in the reaction solution by changing a magnetic field that is exerted on the magnetic particles.

2. The method according to Claim 1,
wherein the step (A1) further comprises stirring or shaking the reaction solution containing the substance of interest and the magnetic particles.

3. A method for measuring the substance of interest, the method comprising:
a step (a1) of forming the conjugates by the method according to Claim 1 or 2; and
a step (b1) of determining a quantity of the substance of interest by determining a quantity of the magnetic particles that form the conjugates.

4. A kit that is used in the method according to Claim 1 or 2, the kit comprising:
magnetic particles to which a specific binding substance that has an ability to specifically bind to a substance of interest has been bound;
a reaction container in which the substance of interest and the magnetic particles are reacted; and
a magnet.

5. A kit that is used in the method according to Claim 1 or 2, the kit comprising:
magnetic particles to which a specific binding substance that has an ability to specifically bind to a substance of interest can be bound;
a reaction container in which the substance of interest and the magnetic particles are reacted; and
a magnet.

6. A method for forming a conjugate of a substance of interest, a magnetic particle to which a first specific binding substance that has an ability to specifically bind to the substance of interest has been bound, and a second specific binding substance that has an ability to specifically bind to the substance of interest and has been bound to an inner wall of a reaction container, the method comprising:
a step (A2) of performing a binding reaction between the substance of interest and the magnetic particle and a binding reaction between the substance of interest and the second specific binding substance in a reaction solution accommodated in the reaction container and containing the substance of interest and the magnetic particles, and
increasing momentum of the magnetic particles in the reaction solution by changing a magnetic field that is exerted on the magnetic particles.

7. The method according to Claim 6,
wherein the step (A2) further comprises stirring or shaking the reaction solution.

8. A method for measuring the substance of interest, the method comprising:
a step (a2) of forming the conjugates by the method according to Claim 6 or 7; and
a step (b2) of determining a quantity of the substance of interest by determining a quantity of the magnetic particles that form the conjugates.

9. A kit that is used in the method according to Claim 6 or 7, the kit comprising:
magnetic particles to which a first specific binding substance that has an ability to specifically bind to a substance of interest has been bound;
a reaction container in which a second specific binding substance that has an ability to specifically bind to the substance of interest has been bound to an inner wall; and
a magnet.

10. A kit that is used in the method according to Claim 6 or 7, the kit comprising:
magnetic particles to which a first specific binding substance that has an ability to specifically bind to a substance of interest can be bound;
a reaction container in which a second specific binding substance that has an ability to specifically bind to the substance of interest has been bound to an inner wall; and
a magnet.
